# FASCICULE DE BREVET EUROPEEN

(11) **EP 2 427 139 B1**
(45) Date de publication et mention de la délivrance du brevet: **04.12.2019**
(21) Numéro de dépôt: 10727100.9
(22) Date de dépôt: 05.05.2010
(51) Int. Cl.: A61F 2/01

(54) **KIT D'EXTRACTION POUR FILTRE POUR VEINE CAVE**
EXTRAKTIONSKIT FÜR EINEN VENA-CAVA-FILTER
EXTRACTION KIT FOR A FILTER FOR THE VENA CAVA

(30) Priorité: 06.05.2009 FR 0953017
(43) Date de publication de la demande: 14.03.2012
(73) Titulaire: A.L.N., 20240 Ghisonaccia (FR)
(72) Inventeur: NIGON, Alain, F-83230 Bormes Les Mimosas (FR)
(74) Mandataire: Plasseraud IP
(86) Numéro de dépôt international: PCT/FR2010/050857
(87) Numéro de publication internationale: WO 2010/128249

(56) Documents cités:
- WO-A-00/44308
- WO-A-01/13984
- WO-A-2005/120627
- WO-A1-00/18467
- WO-A2-2007/079415
- US-A1- 2006 211 979
- US-A1- 2007 167 974
- US-A1- 2007 265 595
- US-B1- 6 846 317

## Description

La présente invention concerne un kit d'extraction pour filtre pour veine cave (appelé encore "filtre-cave"), son utilisation et des kits le renfermant.

Certaines pathologies peuvent avoir pour conséquence une coagulation excessive du sang, augmentant la tendance de celui-ci à former des caillots (ou thrombus). Il s'ensuit alors une thrombose veineuse profonde.

Un risque majeur survient chez un patient souffrant de thrombose veineuse profonde quand un caillot se détache des membres inférieurs et se déplace à travers la veine cave inférieure pour obstruer une ou plusieurs artères pulmonaires. Les poumons n'étant plus alimentés en oxygène, l'embolie pulmonaire, souvent mortelle se produit.

Des malades présentent des contre-indications formelles à l'institution d'un traitement anticoagulant.

Les filtres pour veine cave sont conçus pour protéger efficacement notamment de tels malades contre les risques d'embolie pulmonaire.

Un filtre pour veine cave est un dispositif mécanique en forme d'ombrelle, introduit dans une veine cave par cathéter. Le filtre permet d'arrêter la migration des caillots provenant des veines des membres inférieurs.

Il peut être installé de façon définitive. Mais dans de nombreux cas, comme lorsque le risque d'embolie pulmonaire est acceptable ou qu'il n'y a plus de risque d'embolie pulmonaire clinique, ou lorsque le patient ne nécessite plus de traitement contre la thrombose veineuse profonde, ou qu'un traitement par anticoagulants est possible, en cas de changement de position ou de perte d'intégrité du filtre, il est nécessaire de pouvoir l'extraire.

EP-A-1 187 578 décrit un kit pour le retrait d'un filtre pour vaisseau sanguin du type parapluie formé d'une ogive servant de manchon de retenue à une pluralité de brins élastiques naturellement écartés les uns des autres et terminés en crochets dirigés vers l'extérieur pour se bloquer sur la paroi d'un vaisseau comprenant un premier cathéter, une tige apte à s'insérer dans le cathéter interne et comportant à une de ses extrémités une pluralité de branches élastiques s'évasant à partir de la tige, naturellement écartées les unes des autres et terminées en crochets dirigés vers l'intérieur pour agripper l'ogive du filtre en se resserrant et optionnellement un second cathéter (cathéter externe), de diamètre apte à être glissé le long du cathéter interne et un troisième cathéter, de diamètre adapté à l'introduction dans le cathéter externe, dont l'extrémité avant est fermée et émoussée pour servir de dilatateur lors de l'introduction dans un vaisseau de l'ensemble constitué par le second et le troisième cathéter.

Une complication de l'utilisation des filtres est le basculement ("tilting") du filtre dans la veine. L'ogive vient se caler contre la paroi de la veine. Ce basculement rend difficile le retrait du filtre.

US 2006/211797 décrit un cathéter à flexibilité variable capable d'avancer jusqu'à l'endroit traité avec suffisamment de souplesse à travers un chemin tortueux mais peut aussi avoir une rigidité suffisante pour avancer au travers d'une occlusion artérielle. Ce cathéter présente une rigidité qui peut être contrôlée pendant l'utilisation. Il comprend un dispositif chauffant et un liant fusible en contact thermique avec le dispositif chauffant, le dispositif rigidifiant étant disposé dans la gaine. Le liant est substantiellement solide à la température du corps et peut se ramollir au-dessus de sa température de transition.

La présente demande a pour objet des perfectionnements de l'organe utilisé pour agripper l'ogive d'un filtre pour vaisseau sanguin du type parapluie en vue de son retrait. Par exemple dans EP-A-1 187 578, l'organe en question est la tige comportant à une de ses extrémités une pluralité de branches élastiques. Elle a aussi pour objet des simplifications et de ce fait plus simple que des dispositifs actionnables par un câble.

La présente demande a donc pour objet un organe de retrait pour filtre pour veine cave essentiellement constitué d'une tête et de brins à mémoire de forme ou façonnés formant une queue, lesdits brins étant fixés à la tête et situés du même côté par rapport à la tête, ledit organe de retrait comprenant une tige comportant à son extrémité distale une pluralité de branches élastiques s'évasant à partir de la tige, naturellement écartées les unes des autres et terminées en crochets dirigés vers l'intérieur (c'est-à-dire vers l'axe) pour agripper l'ogive du filtre en se resserrant, caractérisé en ce qu'il comprend à proximité de l'extrémité distale de la tige une zone coudée flexible.

Dans la présente demande et dans ce qui suit, un filtre pour veine cave étant destiné à être placé centré dans une veine, les positions seront indiquées en prenant comme référence l'axe central d'une veine, la paroi vasculaire indiquant donc l'extérieur. Le terme «hors contrainte» désigne la conformation spatiale naturelle de l'objet considéré. Le terme « sous contrainte» désigne la conformation spatiale de l'objet considéré installé en position fonctionnelle dans un vaisseau, ou dans un cathéter, donc soumis à des forces externes. Classiquement, le terme « distal» désigne la partie d'un objet la plus éloignée de l'utilisateur et « proximal» la plus proche.

Le présent organe de retrait n'est pas à flexibilité variable. Il comprend une zone coudée de flexibilité essentiellement constante que l'on soit à température ambiante ou à température du corps humain (environ 37°C) ou quelques degrés de plus que la température du corps humain (par exemple jusque environ 55°C, jusque environ 50°C, ou jusque environ 45°C). Il ne comprend pas de dispositif chauffant ni de liant fusible en contact thermique avec le dispositif chauffant disposés dans une gaine, le liant étant substantiellement solide à la température du corps et pouvant se ramollir au-dessus de sa température de transition.

WO 00/44308 et WO 01/13984 concernent un stent. US 2007/265595 et WO 2005/120627 concernent un cathéter actionnable par un câble

Un dispositif tel que celui du préambule de la revendication 1 est connu du document US-A-6 846 317.

Ledit organe de retrait comprend principalement au bout de son extrémité distale une pluralité de branches élastiques, puis une partie allongée de la tige, et du côté proximal une pièce de manipulation permettant à l'opérateur de saisir notamment entre deux doigts cette extrémité de l'organe de retrait.

La pièce de manipulation comprend de préférence du côté distal un embranchement Luer lock (cône standard Luer à vissage) femelle.

Dans des conditions préférentielles de mise en oeuvre de l'invention, la zone coudée flexible est prévue à moins de 4 cm de l'extrémité distale de la tige hors branches élastiques, avantageusement de 1 à 3, notamment de 1 à 2, tout particulièrement de 1,5 à 2 cm de ladite extrémité.

A cause de la zone coudée, l'axe de l'extrémité distale de la tige forme un angle par rapport à l'axe principal (l'axe de la plus grande longueur droite) de la tige. Hors contrainte, la zone coudée peut former un angle de 110° à 170°, de préférence de 120° à 160°, notamment de 130° à 150°, tout particulièrement d'environ 150°.

La longueur de la zone coudée peut aller de 2 à 15, de préférence de 2 à 12, notamment de 5 à 11, tout particulièrement de 5 à 10 mm.

La zone coudée est flexible. On entend par là que hors contrainte, la zone coudée forme un angle différent de 180°, mais si l'on fait glisser sur l'extrémité distale de l'organe de retrait un cathéter conventionnel pour ce genre d'intervention, particulièrement un cathéter 6F, 7F, 8F ou 9F, l'action du cathéter suffit pour redresser la zone coudée pour conférer à l'organe de retrait une forme rectiligne.

Dans d'autres conditions préférentielles de mise en oeuvre de l'invention, les branches élastiques sont fixées, et notamment serties, sur ou dans un tube distal constituant une partie distale de la tige. Avantageusement, la zone coudée débute à l'autre extrémité dudit tube distal.

Ce tube distal peut avoir une longueur de 9 à 16, de préférence de 10 à 15, notamment de 11 à 14, tout particulièrement de 12 à 13 mm.

Dans encore d'autres conditions de mise en oeuvre de l'invention, la zone coudée est prolongée du côté proximal par un second tube (tube proximal).

Ce tube proximal peut avoir une longueur de 9 à 16, de préférence de 10 à 15, notamment de 11 à 14, tout particulièrement de 12 à 13 mm.

Le tube distal ou le tube proximal auront un diamètre de 1 à 4, de préférence de 1 à 3, notamment de 1 à 2, tout particulièrement d'environ 2,0 mm.

Selon le mode de réalisation de l'organe de retrait de l'invention, la partie allongée de la tige est fixée au tube proximal ou au tube distal ou aux deux. Elle peut être par exemple collée, soudée, brasée ou notamment sertie. Ladite partie allongée de la tige peut être formée de plusieurs brins ou de préférence d'un brin unique.

La zone coudée peut être réalisée par exemple en matière plastique polymère. Elle est avantageusement réalisée en fil métallique. On peut alors trouver par exemple 1, 2, 3, 4 ou 5 fils métalliques. On préfère 1 ou 2 fils métalliques notamment en acier inox, particulièrement de diamètre environ 0,3 mm. La forme coudée de chaque fil peut être conférée avant ou après solidarisation aux tubes.

Le fil métallique ou un fil métallique peut être notamment un rétrécissement de la tige pour augmenter la flexibilité à ce niveau ou la tige elle même, particulièrement pour un organe de retrait comprenant un tube distal et mais pas de tube proximal.

Le fil métallique formant la tige a un diamètre de 0,3 à 0,9, de préférence de 0,4 à 0,8, notamment de 0,5 à 0,7, tout particulièrement d'environ 0,5 mm.

Dans des conditions préférentielles de mise en œuvre de l'invention, l'organe de retrait comprend du côté distal un tube distal et un tube proximal, de préférence de même diamètre ou de diamètres proches.

Dans ce cas, avantageusement la zone coudée flexible a un diamètre identique à celui des tubes distal et proximal. On peut alors prévoir une gaine, par exemple en matière plastique polymère notamment entourant les 1, 2, 3, 4 ou 5 fils métalliques précités pour constituer avec les tubes une zone de même diamètre. Ainsi, lorsque l'on fait progresser le cathéter adapté sur l'organe de retrait vers l'extrémité distale, il n'y a aucun risque de blocage du cathéter contre le chant du tube distal.

Dans ce but, on peut aussi par exemple prévoir que les extrémités proximales des tubes distal et proximal ou du tube distal soient biseautées ou arrondies ou soient munies d'un revêtement biseauté pour former un chanfrein.

Les branches élastiques fixées, et notamment serties, sur ou dans un tube constituant une partie distale de la tige peuvent être au nombre de 5 à 8, notamment de 6 à 7, tout particulièrement au nombre de 6.

De préférence l'extrémité distale d'au moins deux des branches élastiques est décalée longitudinalement. Notamment la longueur libre des branches élastiques est inégale pour toutes les branches élastiques.

Les branches élastiques sont avantageusement réalisées en métal comme en phinox, en alliage nitinol (NiTi), en titane et particulièrement en inox.

Réalisées en inox, au nombre de 5 à 8 et notamment de 6 ou 7, elles auront un diamètre de préférence de 0,2 à 0,6, notamment de 0,3 à 0,5, tout particulièrement d'environ 0,4 mm.

La longueur des branches élastiques mesurée entre le crochet et la base libre de la branche est avantageusement de 1,0 à 5,0, de préférence de 1,5 à 4,0, notamment de 1,5 à 3,0, tout particulièrement de 1,9 à 2,2 cm.

Les branches élastiques terminées en crochets dirigés vers l'intérieur pour agripper l'ogive du filtre en se resserrant. A cette fin, l'extrémité distale de chaque branche élastique est de préférence repliée en L pour former un angle approximativement droit par rapport à la longueur de la branche.

Compte tenu de son utilisation, les matériaux utilisés pour la fabrication de l'organe de retrait sont des métaux, des composites ou polymères biocompatibles et/ou bio-implantables.

La présente demande a aussi pour objet un procédé de fabrication de certains organes de retrait ci-dessus caractérisé en ce que l'on prévoit un premier tube auquel on fixe axialement à une extrémité, de préférence par sertissage, une pluralité de branches élastiques s'évasant à partir de la tige, naturellement écartées les unes des autres et terminées en crochets dirigés vers l'intérieur, fixe axialement à l'autre extrémité, de préférence par sertissage, 1, 2, 3, 4 ou 5 fils métalliques que l'on fixe axialement à une extrémité, de préférence par sertissage, d'un second tube pour former une zone coudée flexible, et fixe axialement à l'autre extrémité, de préférence par sertissage, une partie allongée de la tige, et si désiré, du côté proximal de ladite tige, une pièce de manipulation permettant à l'opérateur de saisir notamment entre deux doigts cette extrémité de l'organe de retrait.

Les organes de retrait objet de la présente invention possèdent de très intéressantes propriétés. Ils permettent de récupérer des filtres pour veine cave bien axés dans une veine, mais en outre, grâce à leur conception, le même organe de retrait permet de récupérer facilement des filtres qui ont basculé dans la veine, et de les redresser si nécessaire. Il est aussi bien utilisable pour un filtre comportant une simple ogive, qu'une ogive munie d'un anneau ou d'un crochet proximal. La configuration particulière de l'extrémité de l'organe de retrait avec, à proximité de l'extrémité distale de la tige, une zone coudée fait que certains des crochets se trouvent naturellement placés contre la paroi d'un tube comme un vaisseau sanguin, sans intervention autre, pour crocheter l'ogive d'un filtre basculé. La flexibilité de la zone coudée permet à l'organe de retrait d'avancer jusqu'à l'emplacement d'un filtre avec suffisamment de souplesse à travers un chemin tortueux mais l'ensemble de l'organe de retrait a une rigidité suffisante pour avancer, par exemple au travers d'une occlusion artérielle.

Ces qualités sont illustrées ci-après dans la partie descriptive des figures. Elles justifient l'utilisation des organes de retrait ci-dessus décrits, dans une méthode de retrait ou de redressement d'un filtre pour veine cave installé dans une veine.

C'est pourquoi la présente demande a aussi pour objet une méthode de retrait ou de redressement d'un filtre pour veine cave installé dans une veine caractérisé en ce que l'on insère un organe de retrait ci-dessus décrit en position rectiligne dans la veine cave d'un patient et agrippe l'ogive d'un filtre pour veine cave, soit directement si le filtre est correctement axé, soit après avoir reculé le cathéter maintenant l'organe de retrait en position rectiligne pour incurver son extrémité distale.

La présente demande a aussi pour objet un kit (ou ensemble) comprenant
- un organe de retrait ci-dessus décrit,
- un ensemble cathéter interne de préférence 7F, cathéter externe de préférence 9F, de diamètres adaptés à l'organe de retrait, le cathéter interne et le cathéter externe étant de préférence de diamètre différant de 2F (par exemple cathéter interne 7F et cathéter externe 9F).

La présente demande a également pour objet un kit ci-dessus décrit, comprenant en outre un cathéter dilatateur et optionnellement un guide en forme de J pour arriver jusqu'au site d'implantation.

Elle a de plus pour objet un des kits ci-dessus, renfermant en outre des instructions pour l'utilisation du matériel du kit.

Elle a encore pour objet un des kits ci-dessus, renfermant en outre une aiguille de ponction.

Les organes de retrait et les kits selon l'invention peuvent plus précisément être utilisés comme suit, dans le cas d'une veine cave inférieure :
On ponctionne la veine jugulaire droite à l'aide d'une aiguille de ponction. On fait descendre un guide en forme de J recouvert de TEFLON® à l'aide d'un raidisseur jusqu'à 5 cm au-dessus du filtre à extraire, en suivant sa progression par radioscopie. On retire l'aiguille de ponction. On fait glisser sur un guide en forme de J l'ensemble du cathéter externe et du cathéter dilatateur jusqu'à sa partie distale en suivant sa progression par radioscopie grâce à une bague radio-opaque. On retire en même temps le guide en J et le cathéter dilatateur sous radioscopie de face et surtout de profil. On installe une cale entre la pièce de manipulation de l'organe de retrait et l'entrée du cathéter interne pour éviter de repousser par inadvertance la tige et ouvrir en parapluie la pince à crochets. On introduit le cathéter interne dans lequel est installé l'organe de retrait en s'assurant de ne pas faire descendre celui-ci au-delà de 5 cm de garde prévue et en continuant à suivre par radioscopie toute la manipulation. On ôte alors la cale et on avance un peu la tige pour ouvrir la pince à crochets. Une fois les crochets récupérateurs ouverts, on descend lentement au-dessus de l'ogive du filtre à récupérer. On s'assure que les crochets se placent suffisamment en dessous de l'ogive. Si le filtre est désaxé, on recule un peu le cathéter externe vers la partie proximale de l'organe de retrait, ce qui provoque la courbure de l'extrémité distale. On peut ainsi mieux agripper l'ogive du filtre à récupérer.

A ce moment de la procédure, on fait glisser lentement le cathéter externe sur les crochets à l'extrémité de la tige. On bloque éventuellement en installant à nouveau la cale ou une cale un peu plus courte. Toujours sous radioscopie, on s'assure que les crochets de la pince sont bien refermés sous l'ogive du filtre et bien centrés. On continue à descendre lentement le cathéter externe au-delà de l'ogive du filtre, de façon à en replier les brins vers l'intérieur. On s'assure que les crochets du filtre sont bien décrochés de la paroi vasculaire. D'un mouvement lent, on pousse le cathéter externe le long du cathéter interne jusqu'à ce que la pince et le filtre qu'elle a accroché soient entièrement introduits dans le cathéter externe. On ressort l'ensemble des deux cathéters avec l'organe de retrait et le filtre. On assure alors l'hémostase au point de ponction.

On comprend de ce qui précède que le cathéter externe peut avancer au-delà de l'extrémité du cathéter interne

Les conditions préférentielles de mise en oeuvre des organes de retrait selon l'invention ci-dessus décrites s'appliquent également aux autres objets de l'invention visés ci-dessus, notamment aux procédés pour leur fabrication, aux kits et procédés d'utilisation.

L'invention sera mieux comprise si l'on se réfère aux dessins annexés sur lesquels
- la figure 1 représente une vue de côté de l'extrémité distale d'un organe de retrait selon l'invention, en coupe partielle au niveau du coude, hors contrainte ;
- la figure 2 représente une variante de réalisation de la figure 1 ;
- la figure 3 est une vue en élévation latérale et en coupe partielle d'un kit mettant en oeuvre l'organe de retrait de l'invention ; et
   la figure 4 représente également une vue en élévation latérale et en coupe partielle d'un kit mettant en œuvre l'organe de retrait de l'invention.
- la figure 5 représente une vue de côté de l'extrémité proximale d'un organe de retrait perfectionné selon l'invention.

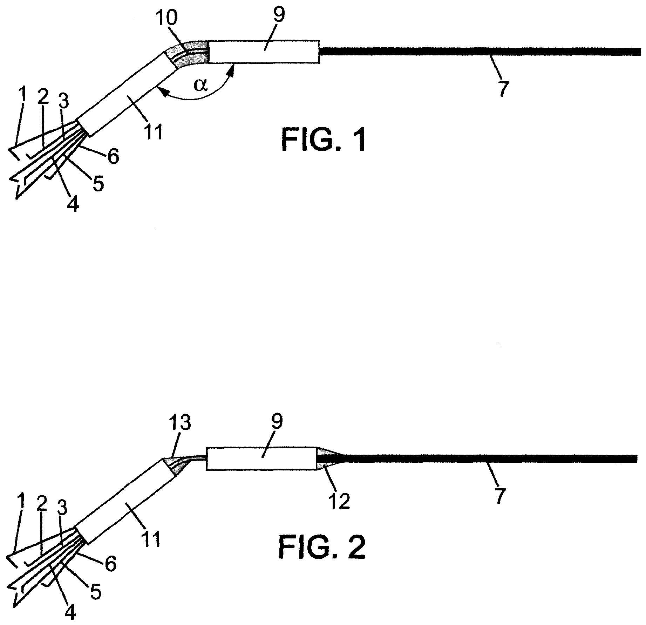

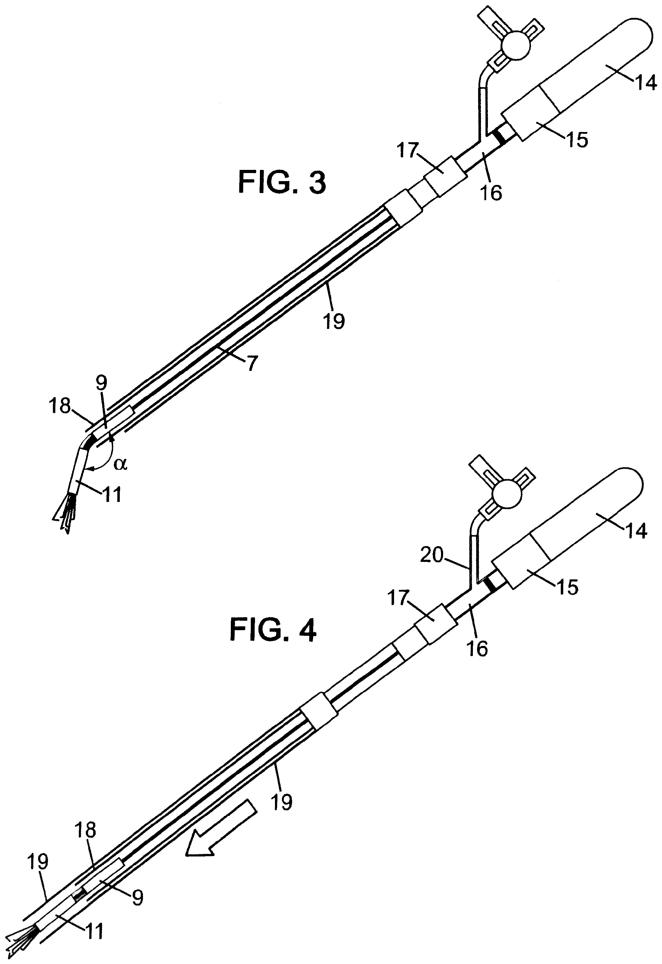

Sur la figure 1 on distingue l'extrémité distale d'un organe de retrait de l'invention en coupe partielle au niveau du coude. Un tel organe de retrait comprend au bout de son extrémité distale une pluralité de branches élastiques 1, 2, 3, 4, 5, 6, puis une partie allongée de la tige 7 et du côté proximal une pièce de manipulation 14 représentée sur la figure 3. En partant du côté proximal, on observe la partie allongée de la tige qui est ici formée d'un brin en acier unique de diamètre 0,7 mm. Celui-ci est serti dans un tube proximal 9 en acier de diamètre 2 mm et de longueur 12 mm. A l'autre extrémité du tube 9, sont sertis deux brins 10 en acier, coudés, dont l'autre extrémité est sertie dans un tube distal 11 également de diamètre 2 mm et de longueur 12 mm. Dans la partie distale du tube distal 11, sont serties six branches élastiques 1, 2, 3, 4, 5, 6 réalisées en fil d'acier de diamètre 0,4 mm. Ces branches ont une forme générale de L dont l'angle formé par la petite barre est de 90° par rapport à la grande barre.

Ces branches sont de longueur inégale. On trouve une branche de longueur 19,75mm, une branche de longueur 20,07mm, une branche de longueur 20,21mm, une branche de longueur 20,47mm, une branche de longueur 20,68mm et une branche de longueur 20,99mm.

Les petites barres formant le crochet ont une longueur d'environ 1,9 mm.

Les longueurs libres des branches (c'est-à-dire la longueur allant de la sortie du tube distal à la partie la plus distale des branches) s'échelonnent donc entre environ 19 et environ 21 mm.

Les brins 10 sont incorporés dans une gaine de matériau polymère de même diamètre que celui des tubes proximal 9 et distal 11.

De par la présence des brins coudés 10, l'axe du tube distal et l'axe du tube proximal forment l'un par rapport à l'autre un angle α d'environ 150°.

Sur la figure 2 on observe une variante de la figure 1 dans laquelle, pour éviter que lors de la progression d'un cathéter vers l'extrémité distale celui-ci se bloque en butée contre le champ d'un des tubes, l'extrémité proximale de chacun de ceux-ci est chanfreinée en forme de cône 12, 13. Ce dernier a été formé en chanfreinant les tubes.

Dans une variante de réalisation avec chanfrein, on a installé une pièce tronconique rapportée, en matière plastique polymère, pour constituer le chanfrein.

Dans une autre variante de réalisation avec chanfrein, on a installé uniquement le tube distal 11, et c'est la tige 7 qui pénètre directement dans ce tube distal 11. On a dans ce cas rétréci la tige 7 au niveau du coude, à un diamètre de 0,5 mm, pour augmenter la flexibilité à ce niveau.

Dans encore une autre variante de réalisation, on a utilisé pour la réalisation de la tige 7 un fil en acier inox de diamètre de 0,5 mm.

Sur la figure 3 on observe au centre du montage l'organe de retrait selon l'invention. A l'extrémité proximale de la tige 7, on trouve une pièce de manipulation 14 en matière plastique rigide. Celle-ci est cylindrique et terminée du côté proximal par un dôme hémisphérique et du côté distal par un emmanchement Luer Lock mâle 15. Ce dernier s'introduit et se bloque sur l'extrémité proximale d'un raccord 16 en Y, lui-même muni à son extrémité distale d'un emmanchement Luer Lock mâle 17 analogue à celui de la poignée. Le cône de ce dernier emmanchement 17 s'introduit dans l'extrémité du Luer Lock femelle du cathéter interne 18. Une valve hémostatique est prévue du côté distal de la branche principale du raccord en Y pour empêcher le sang de remonter au niveau de la poignée. Un robinet 3 voies muni de deux Luer Lock femelles est relié à la branche secondaire 20 du raccord en Y. A la sortie distale de ce cathéter interne 18 (diamètre 7F= 2,31 mm interne), on trouve le tube proximal 9 de l'organe de retrait. La partie distale de ce dernier n'étant soumise à aucune contrainte, elle se trouve coudée selon un angle α d'environ 150°.

Sur la figure 4 on distingue les mêmes éléments que sur la figure 3. Mais le cathéter externe 19 (diamètre 9F= 3 mm interne) a été avancé et, grâce à sa flexibilité, l'extrémité distale de l'organe de retrait a été rendue rectiligne.

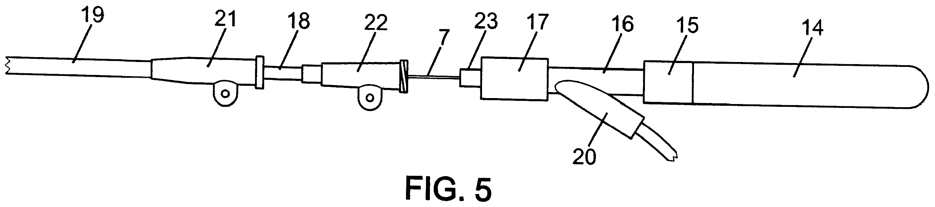

Sur la figure 5 on distingue, depuis l'extrémité proximale à droite du dessin, une pièce de manipulation 14 en matière plastique rigide solidaire de la tige 7. Cette pièce de manipulation 14 est cylindrique et terminée du côté proximal par un dôme hémisphérique et du côté distal par un emmanchement Luer Lock mâle 15. Ce dernier coopère avec un cône Luer Lock femelle du raccord 16 en Y. A l'autre extrémité de la branche principale du raccord 16 en Y on trouve un emmanchement Luer Lock mâle 17, analogue à celui 15 de la poignée, dont on peut observer le cône 23. Le raccord 16 en Y est traversé par la tige 7 qui peut coulisser dans le cathéter interne 18 (ici de diamètre 7F), qui lui-même peut coulisser dans le cathéter externe 19 (ici de diamètre 9F).

L'extrémité proximale du cathéter interne 18 comporte un surmoulage 22 dont la lumière proximale est un cône Luer femelle, et l'anneau proximal permet le blocage (Lock). Ce surmoulage 22 est muni d'une oreille percée, comme d'ailleurs le surmoulage 21 dont est munie l'extrémité proximale du cathéter externe 19. La lumière proximale du surmoulage 21 est agencée pour coopérer avec l'extrémité distale du surmoulage 22.

## Revendications

1. Un organe de retrait pour filtre pour veine cave essentiellement constitué d'une tête et de brins à mémoire de forme ou façonnés formant une queue, lesdits brins étant fixés à la tête et situés du même côté par rapport à la tête, ledit organe de retrait comprenant une tige (7) comportant à son extrémité distale une pluralité de branches élastiques (1,2,3,4,5,6) s'évasant à partir de la tige (7), naturellement écartées les unes des autres et terminées en crochets dirigés vers l'intérieur pour agripper l'ogive du filtre en se resserrant, **caractérisé en ce qu'**il comprend à proximité de l'extrémité distale de la tige (7) une zone flexible qui est coudée hors contrainte.

2. Un organe de retrait selon la revendication 1, **caractérisé en ce que** la zone coudée flexible est prévue de 1 à 3 cm de l'extrémité distale de la tige hors branches élastiques.

3. Un organe de retrait selon la revendication 1 ou 2, **caractérisé en ce que** l'axe de l'extrémité distale de la tige (7) forme un angle (α) de 120° à 160° par rapport à l'axe principal de la tige (7).

4. Un organe de retrait selon l'une des revendications 1 à 3, **caractérisé en ce que** la longueur de la zone coudée flexible est de 5 à 11 mm.

5. Un organe de retrait selon l'une des revendications 1 à 4, **caractérisé en ce que** les branches élastiques (1,2,3,4,5,6) sont fixées sur ou dans un tube distal (11) constituant une partie distale de la tige (7).

6. Un organe de retrait selon la revendication 5, **caractérisé en ce que** la zone coudée est prolongée du côté proximal par un second tube (9) (tube proximal).

7. Un organe de retrait selon l'une des revendications 5 et 6, **caractérisé en ce que** le tube distal (11) ou le tube proximal (9) a un diamètre de 1 à 4 mm.

8. Un organe de retrait selon l'une des revendications 5 à 7, **caractérisé en ce que** l'organe de retrait comprend du côté distal un tube distal (11) et un tube proximal (9) de mêmes diamètres.

9. Un organe de retrait selon l'une des revendications 5 à 8, **caractérisé en ce que** les branches élastiques (1,2,3,4,5,6) fixées sur ou dans un tube (11) constituant une partie distale de la tige (7) sont au nombre de 5 à 8 et l'extrémité distale d'au moins deux des branches élastiques (1,2,3,4,5,6) est décalée longitudinalement.

10. Un ensemble comprenant :
- un organe de retrait tel que défini à l'une des revendications 1 à 9,
- un ensemble cathéter interne, cathéter externe, de diamètres adaptés à l'organe de retrait.

## Patentansprüche

1. Rückzugselement für einen Filter für eine Hohlvene, welches im Wesentlichen aus einem Kopf und Gedächtnisdrähten von der Form eines oder einen Stab ausbildend gebildet ist, wobei die Drähte an dem Kopf befestigt und an derselben Seite bezüglich des Kopfs platziert sind, wobei das Rückzugselement eine Stange (7) umfasst, welche an ihrem distalen Ende eine Mehrzahl von elastischen Zweigen (1, 2, 3, 4, 5, 6) umfasst, welche sich ausgehend von der Stange (7) aufweiten, natürlich voneinander beabstandet und in Haken endend, welche zu dem Innenraum gerichtet sind, um den Bogen des Filters bei einem Zusammenziehen zu greifen, **dadurch gekennzeichnet, dass** es in der Nähe des distalen Endes der Stange (7) eine flexible Zone umfasst, welche unbelastet gebogen ist.

2. Rückzugselement nach Anspruch 1, **dadurch gekennzeichnet, dass** die flexible gebogene Zone in 1 bis 3 cm von dem distalen Ende der Stange ohne elastische Zweige vorgesehen ist.

3. Rückzugselement nach Anspruch 1 oder 2, **dadurch gekennzeichnet, dass** die Achse des distalen Endes der Stange (7) einen Winkel (α) von 120° bis 160° bezüglich der Hauptachse der Stange (7) bildet.

4. Rückzugselement nach einem der Ansprüche 1 bis 3, **dadurch gekennzeichnet, dass** die Länge der flexiblen gebogenen Zone von 5 bis 11 mm beträgt.

5. Rückzugselement nach einem der Ansprüche 1 bis 4, **dadurch gekennzeichnet, dass** die elastischen Zweige (1,2, 3, 4, 5, 6) an oder in einem distalen Rohr (11) befestigt sind, welches einen distalen Teil des Stange (7) bildet.

6. Rückzugselement nach Anspruch 5, **dadurch gekennzeichnet, dass** die gebogene Zone von der proximalen Seite durch ein zweites Rohr (9) (proximales Rohr) verlängert ist.

7. Rückzugselement nach einem der Ansprüche 5 und 6, **dadurch gekennzeichnet, dass** das distale Rohr (11) oder das proximale Rohr (9) einen Durchmesser von 1 bis 4 mm aufweist.

8. Rückzugselement nach einem der Ansprüche 5 bis 7, **dadurch gekennzeichnet, dass** das Rückzugselement an der distalen Seite ein distales Rohr (11) und ein proximales Rohr (9) von gleichen Durchmessern umfasst.

9. Rückzugselement nach einem der Ansprüche 5 bis 8, **dadurch gekennzeichnet, dass** die elastischen Zweige (1,2, 3, 4, 5, 6), welche an oder in dem Rohr (11) befestigt sind, welches einen distalen Teil der Stange (7) bildet, von der Anzahl von 5 bis 8 sind, und das distale Ende von wenigstens zwei der elastischen Zweige (1, 2, 3, 4, 5, 6) longitudinal verlagert ist.

10. Anordnung, umfassend:
- ein Rückzugselement wie in einem der Ansprüche 1 bis 9 definiert,
- eine Anordnung aus einem internen Katheder, einem externen Katheder mit Durchmessern, welche auf das Rückzugselement angepasst sind.

## Claims

1. A withdrawal member for a filter for the vena cava, essentially consisting of a head and shape memory or shaped strands forming a tail, said strands being fixed to the head and located on the same side of the head, said withdrawal member comprising a rod (7) having at its distal end a plurality of resilient arms (1,2, 3, 4, 5, 6) flaring from the rod (7), naturally spaced apart from each other and ending in hooks directed toward the inside so as to grip the cap of the filter upon tightening, **characterized in that** it comprises in proximity to the distal end of the rod (7) a flexible region which is bent without stress.

2. The withdrawal member as claimed in claim 1, **characterized in that** the flexible bent region is provided at from 1 to 3 cm from the distal end of the rod without resilient arms.

3. The withdrawal member as claimed in claim 1 or 2, **characterized in that** the axis of the distal end of the rod (7) forms an angle (α) of from 120° to 160° with respect to the principal axis of the rod (7).

4. The withdrawal member as claimed in one of claims 1 to 3, **characterized in that** the length of the flexible bent region is from 5 to 11 mm.

5. The withdrawal member as claimed in one of claims 1 to 4, **characterized in that** the resilient arms (1, 2, 3, 4, 5, 6) are fixed on or in a distal tube (11) constituting a distal part of the rod (7).

6. The withdrawal member as claimed in claim 5, **characterized in that** the bent region is extended on the proximal side by a second tube (9) (proximal tube).

7. The withdrawal member as claimed in one of claims 5 and 6, **characterized in that** the distal tube (11) or the proximal tube (9) has a diameter of from 1 to 4 mm.

8. The withdrawal member as claimed in one of claims 5 to 7, **characterized in that** the withdrawal member comprises on the distal side a distal tube (11) and a proximal tube (9) having the same diameters.

9. The withdrawal member as claimed in one of claims 5 to 8, **characterized in that** the number of resilient arms (1, 2, 3, 4, 5, 6) fixed on or in a tube (11) constituting a distal part of the rod (7) is from 5 to 8 and the distal end of at least two of the resilient arms (1, 2, 3, 4, 5, 6) is longitudinally offset.

10. An assembly comprising:
- a withdrawal member as defined in one of claims 1 to 9,
- a set consisting of an internal catheter and an external catheter, having diameters adapted to the withdrawal member.
